# EUROPÄISCHE PATENTANMELDUNG

(11) **EP 1 188 548 A2**
(43) Veröffentlichungstag der Anmeldung: **20.03.2002**
(21) Anmeldenummer: 01119994.0
(22) Anmeldetag: 18.08.2001
(51) Int. Cl.: B32B 5/26, B32B 7/04, D04H 13/00, A61L 15/00, A61F 13/00

(54) **Wundauflage und/oder Kompresse aus mehreren Lagen Vliesstoff**

(30) Priorität: 14.09.2000 DE 10045462
(71) Anmelder: Beiersdorf Aktiengesellschaft, 20245 Hamburg (DE); Amoco Deutschland GmbH, 48599 Gronau (DE)
(72) Erfinder: Lenz, Dirk, Dr., 20253 Hamburg (DE)

(57) **Zusammenfassung**

Wundauflage und/oder Kompresse aus einer der Haut zugewandten Lage eines Spinnvliesstoffes sowie mindestens einer auf dem Spinnvliesstoff angeordneten Lage eines Stapelfaservliesstoffes, wobei die Lagen unlösbar durch Ultraschall-Verschweißen miteinander verbunden sind.

## Beschreibung

Die Erfindung betrifft eine Wundauflage und/oder Kompresse aus mehreren Lagen Vliesstoff, insbesondere eine ultraschallverfestigte Wundauflage und/oder Kompresse

Als Wundauflagen und/oder Kompressen für medizinische Zwecke sind zahlreiche Materialien auf Folien-, Gewebe-, Gewirke-, Vlies-, Gel- oder Schaumstoff-Basis bekannt und werden auch in der Praxis eingesetzt. Diese flächigen Malerialien müssen gut saugfähig, hautverträglich, luft- und wasserdampfdurchlässig sowie gut anmodellierbar und anschmiegsam sein. Zur Handhabung und beim Gebrauch sind bei Wundauflagen und Kompressen aber auch eine ausreichende Festigkeit gefordert. Weiterhin sollte das Trägermaterial auch nach dem Durchnässen eine ausreichende Festigkeit und geringe Dehnbarkeit aufweisen.

An eine Wundauflage werden folgende Anforderungen gestellt, die es zu erfüllen gilt:
- Die Wundauflage muß eine gute Saugfähigkeit für Wundflüssigkeit aufweisen, aber gleichzeitig eine ausreichende Naßfestigkeit zeigen.
- Die Wundauflage muß die Wunde zuverlässig vor dem Eindringen von Fremdkörpern schützen, darf allenfalls gering, sollte aber nicht mit der Wunde verkleben.
- Die Wundauflage darf nicht zu Reizzuständen im bedeckten Gewebe führen.
- Die Wundauflage soll sich an konturierten Körperpartien gut anschmiegen.
- Die Wundauflage soll für Gas- und Wasserdampf durchlässig sein.
- Des weiteren soll die Wundauflage erlauben, mit Medikamenten kombiniert zu werden.

In besonderer Weise eignet sich die Materialklasse der Vliesstoffe als Wundauflage oder Kompresse. Sie sind in der Regel luftdurchlässig, bei Verwendung von gängigen Fasermaterialien preiswerter als Gewebe oder Gewirke herzustellen und vermitteln eine hochwertige Haptik.
Nachteilig erweist sich bei den gängigen Vliesstoffen für Pflasteranwendungen, daß sie nur ein bedingt elastisches Verhalten aufweisen und daher nicht optimal für konturierte Körperstellen geeignet sind.

Vliesstoffe können nach vielfältigen Verfahren zum Beispiel nach dem Trockenverfahren dem Spinnvliesverfahren oder Naßverfahren hergestellt werden. Eine Reihe von Veredlungsschritten schließt sich an. Zum Teil werden chemische Bindemittel verwandt (Trockenvliese), die den Nachteil unerwünschter chemischer Stoffe in der Nähe der Wunde bei Anwendung des Pflaster nach sich ziehen.

Die Verfestigung von Vliesstoffen für medizinische Anwendungen in Wundauflagen und Kompressen erfolgt beispielsweise auf thermischen oder mechanischem Wege, so daß der fertige Vliesstoff bei der Herstellung mit keinen weiteren Prozeß- oder Hilfschemikalien in Kontakt kommt. Dadurch zeichnen sich die nach diesem Verfahren, hergestellten Materialien als besonders geeignet für den Einsatz in Medizinprodukten wie zum Beispiel Verbandmaterialien aus.

Herkömmliche Vlies-Wundauflagen und Universal-Kompressen bestehen aus dem Verbund zweier funktionaler Schichten, einer Wundentklebungs-Schicht und einer Verteiler- und/oder Speicher-Schicht.

Die mit der Wunde direkt in Berührung kommende Fläche (primäre Wundauflage) ist im besonderen für die gute Wundverträglichkeit der Wundauflage zuständig. Das Verkleben mit der Wunde nach Blutgerinnung darf nicht auftreten. Diese funktionale Schicht der primären Wundauflage wird von einem speziellen Vliesstoff oder einer perforierten Folie oder einem Gittemetz gebildet. Es dürfen unter keinen Umständen Stoffe aus dieser Schicht in die Wunde gelangen. Das stellt besondere Anforderungen an die Rohstoffauswahl zur Fertigung dieser Schichten, weshalb oft Vliesstoffe oder Folien aus vollsynthetischen, hydrophoben Fasern eingesetzt werden. Andererseits muß der Sekretdurchtritt durch die Wundentklebungsschicht gewährleistet sein.

Die über der primären Wundauflage liegende(n) Schicht(en) sind für die Flüssigkeits- und Sekretaufnahme beziehungsweise dessen Verteilung in der Kompresse/Wundauflage verantwortlich (sekundäre Wundauflage). Hierbei kommen Vliesstoffe zum Einsatz, die hohe interkapillare beziehungsweise intrakapillare Wasseraufnahme zeigen, in der Regel sind diese aus Viskose- oder Baumwollfasem aufgebaut.

Die beiden Schichten sind üblicherweise durch chemische Bindemittel oder Klebstoffe fest miteinander verbunden, was die Gefahr birgt, bei Patienten Reizzustände auszulösen, oder die primäre Wundauflage umhüllt die Absorptionsschicht.

Für die Absorptionsschicht werden vorwiegend Vliesstoffe aus Baumwolle und Viskose eingesetzt. Kompressen und Wundauflagen aus Vliesstoffen mit primärer Wundauflage lassen sich je nach Bedarf zuschneiden und besitzen auch dann noch einen ausreichenden Zusammenhalt beziehungsweise ausreichende Naßfestigkeit.

Tappi J. (1989), 72 (5), 165-70, gibt eine allgemeine Übersicht über die Technologie zur Ultraschallverfestigung von Vliesstoffen:
- Danach ist die Ultraschallverfestigung ein schonender Prozeß.
   Es treten keine hohen Temperaturen auf, die das Material schädigen könnten.
- Die Ultraschallverfahren sind besonders geeignet für Polyolefine, da sie einen relativ niedrigen Schmelzpunkt aufweisen.
- Der Prozeß ist schneller als die thermische Verfestigung
- Das Bindungsverfahren ist geeignet, um besonders "loftige" Materialien miteinander zu verbinden. Es ergibt sich ein weicher Griff bei einem sehr guten Verbund der Schichten.

Aus der US 5,607,798 ist ein Laminat-Aufbau aus drei thermisch zu verfestigten Schichten bekannt, der besteht aus
einem Vliesstoff, der sich aus einer Bikomponentenmischung zusammensetzt, die ihrerseits besteht zu
- 50 bis 95 Gew.-% aus einem isotaktischen Polypropylen und
- 5 bis 50 Gew.-% aus einem statistischen Block-Copolymer aus Propylen und Ethylen, das einen Schmelzpunkt von unter 160 °C aufweist,
einer Zwischenschicht bestehend aus einer Folie oder einem melt-blown Vliesstoff und einem Spinnvliesstoff aus Polypropylen.
Die Verbindung der drei Schichten erfolgt über thermische Bindung, Ultraschall-Verschweißen, Wasserstrahlverfestigung oder Vernadeln.

WO 96/23095 beschreibt den Verfestigungsschritt von Spinnvliesstoffen aus Polypropylen durch Heißkalandrierung, Ultraschallverschweißen oder Heißluft-Verfestigung und Ultraschall. Für die bessere Verarbeitung im "Bonding Process" wird syndiotaktisches Polypropylen zusammen mit isotaktischem Polypropylen eingesetzt, und zwar in einem Verhältnis von 2 bis 15 % zu 85 bis 98 %.
Dadurch sinkt die Verarbeitungstemperatur beim thermischen Verfestigungsprozeß um 10 °F verglichen mit einem ähnlichen thermoplastischen Polypropylen-Vlies ohne Zusatz von syndiotaktischem Polypropylen.

Aufgabe der Erfindung ist es, eine Wundauflage und/oder Kompresse auf Vliesbasis zur Verfügung zu stellen, welche für die genannten Anforderungen hervorragend geeignet sind und die die aus dem Stand der Technik bekannten Nachteile nicht aufweisen.

Gelöst wird die Aufgabe durch ein Vlies, wie es im Anspruch 1 dargelegt ist. Die Untersprüche umfassen vorteilhafte Varianten des Erfindungsgegenstands sowie Verfahren zur Herstellung der Wundauflage und/oder Kompresse.

Demgemäß betrifft die Erfindung eine Wundauflage und/oder Kompresse aus einer der Haut zugewandten Lage eines Spinnvliesstoffes sowie mindestens einer auf dem Spinnvliesstoff angeordneten Lage eines Stapelfaservliesstoffes, wobei die Lagen unlösbar durch Ultraschallverschweißen miteinander verbunden sind.

Derartige Wundauflagen lassen sich durch Vliesstoffe aufbauen, die ein Absorptionsvermögen von 600 bis 1000 g/m² (DIN 53923) gewährleisten. Üblicherweise werden in der Verteiler- oder Speicherschicht Vliesstoffe aus hydrophilen Fasem, wie Viskose oder Baumwolle, und unter einem Zusatz einer Bindefaser (Polyester/Polypropylen) eingesetzt.

In einer bevorzugten Ausführungsform bestehern die Stapelfaservliese und Spinnvliese aus Polypropylen.
Denn für den Fachmann überraschend zeigen Stapelfaservliesstoffe aus reinem Polypropylen, kombiniert mit einem Spinnvliesstoff aus Polypropylen, das für Wundauflagen typische Absorptionsvermögen von > 700 g/m².
Das Absorptionsvermögen beruht auf dem interkapillaren Speichervermögen des Vliesstoffes, d.h. von der dreidimensionalen Anordnung der Fasern im Raum, Art und Stärke der Verdichtung der Fasern.

Für die Wundentklebungsschicht wird vorzugsweise ein Spinnvliesstoff aus Polypropylen mit einem Flächengewicht von ca. 8 bis 25 g/m² eingesetzt.
Für die Verteiler- oder Speicherschicht werden in einer weiteren vorteilhaften Ausführungsform saugfähige Stapelfaservliesstoffe aus insbesondere Polypropylen mit einem Flächengewicht von ca. 15 bis 35 g/m² auswählt.
Vorteilhaft ist, wenn die Anzahl der Lagen Stapelfaservliesstoffe die Zahl fünf nicht übersteigt.

Die Herstellung der Spinn- und Stapelfaservliese erfolgt auf dem Fachmann bekannte Weise.

Ein übliches Verfahren, um die beiden Schichten des Vliesstoffes miteinander zu verbinden, ist die Kalandrierung mittels einer Rasterwalze der beiden Schichten unter Wärmeeinfluß an der Schmelztemperatur des Polymers. Die beiden Vliesstoffe werden an den Rasterpunkten intensiv miteinander verbunden. Auf diese Weise kommt es zu einem guten Verbund, der Basisanforderungen an eine Wundauflage erfüllt.

Nachteile des thermischen Laminierverfahrens sind die Neigung zum Delaminieren beziehungsweise die gegebenenfalls nicht ausreichende Naßfestigkeit.

Ein alternatives kostengünstiges Prinzip zur Herstellung dieser funktionalen Vliesstoff-Wundauflagen und -Kompressen besteht im Verbinden von Stapelfaservliesen und Spinnvliesen aus zum Beispiel aus Polypropylen durch Ultraschall-Verschweißen.

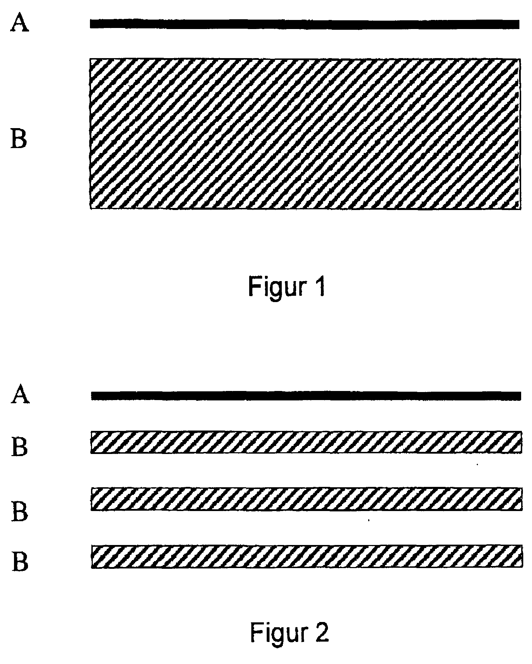

Dabei werden bis zu sieben Schichten aus Hygiene-Standardvliesstoffen miteinander mittels Ultraschall untrennbar verbunden.
Im qualitätsbestimmenden Schritt werden die Lagen der Stapelfaservliesstoffe und die Lage des Spinnvliesstoffes mittels Ultraschall verschweißt. Der Aufbau ist so gewählt, daß die der Wunde zugewandte Seite ein Spinnvlies mit einer glatten Oberfläche aufweist, die die nötige Wundentklebung garantiert. Die darunterliegenden Schichten des Stapelfaservlieses sorgen für die Aufnahme und Speicherung des Wundsekrets (Saugkörper) (s. Fig. 2).

Die Verschweißpunkte können über die Variation der Maske in der Ultraschalleinheit über der Sonotrode variabel gestaltet werden.
Bei einer diagonalen Struktur der Verschweißpunkte sollen diese bevorzugt einen Abstand zwischen 2 und 5 mm in Längs- und Querrichtung des Vliesstoffes aufweisen, insbesondere 3 mm bis 4 mm, weil sich so eine optimale Spaltfestigkeit der Wundauflage/Kompresse ergibt.

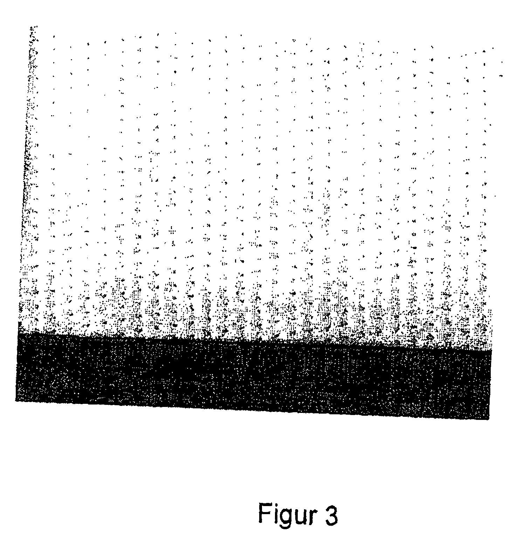

Figur 3 zeigt ein erfindungsgemäßes Material.

Das Verschweißen mittels Ultraschall stellt einen effizienten Prozeß der Laminierung ohne hohe Temperaturen dar, gleichzeitig ist dieser schonend, insbesondere bei der Herstellung von Wundauflagen für labile Ausrüstungen (Wirkstoffe), die bereits in einem der Komponenten vorhanden sind. Schließlich können wesentliche Parameter wie Absorptionsvermögen oder Biegesteifigkeit vorteilhaft beeinflußt werden

Des weiteren sind besondere Ausführungsformen der Wundauflage/Kompresse erhältlich, indem man
- den Spinnvliesstoff mit Wirkstoffen dotiert und/oder
- den Spinnvliesstoff mit bakteriziden und/oder fungizider Stoffen und/oder
- den Spinnvliesstoff mit mikroverkapselten kosmetischen Wirkstoffen ausrüstet.

Die gering saugende Wundentklebungsschicht wird mit dem Wirkstoff ausgerüstet und dann mit den anderen Lagen des Saugkörpers mittels Ultraschall laminiert. Es resultiert ein untrennbarer Verbund aus ausgerüsteter Wundentklebungsschicht und Saugkörper. Der mit Wirkstoff beladene Spinnvliesstoff wird durch den Ultraschallverschweiß-Prozeß nur unwesentlich thermisch belastet. Daher können auch thermisch instabile Formulierungen für diesen Aufbau genutzt werden. Der Wirkstoff befindet sich an der Oberseite der Kompresse/Wundauflage, so daß die Konzentration der wirksamen Substanz gering gehalten werden kann.

Des weiteren umfaßt der Erfindungsgedanke eine Ultraschall verfestigte Wundauflage und/oder Kompresse, die durch Verschweißen einer perforierten Folie (vorzugsweise aus PP) als Wundentklebungsschicht und Standard-Hygienevliesstoffen als Speicherschicht hergestellt wird. Hierbei übemimmt die perforierte Folie die Funktion des Spinnvliesstoffes in der vorbeschriebenen Variante. Die perforierte Folie wird beim Ultraschallverschweißen thermoplastisch mit den Schichten des Saugkörpers verbunden und stellt einen hervorragenden Verbund dar.

Eine weiterhin besonderer Aufbau wird durch Kombination der beiden vorbeschriebenen Varianten erreicht: Es wird eine perforierte Folie, ein Spinnvliesstoff und mehrere Lagen eines saugfähigen Stapelfaservliesstoffes miteinander durch Ultraschallverschweißen verbunden. Folie und Spinnvliesstoff übernehmen die Funktion der Wundentklebungsschicht

Im folgenden soll eine besonders vorteilhafte Ausführung der erfindungsgemäßen Wundauflage mittels mehrerer Figuren beschrieben werden, ohne damit die Erfindung unnötig einschränken zu wollen.

Im einzelnen zeigen zur Verdeutlichung des Aufbaus der vorliegenden Erfindung
- Figur 1: den Aufbau von Vlies-Universalkompressen/Wundauflagen,
- Figur 2: den Schichtenaufbau einer ultraschall-verschweißten Kompresse beziehungsweise Wundauflage,
- Figur 3: eine vorteilhafte Ausführungsform der ultraschall-verfestigten Wundauflage.

Figur 1 zeigt den Aufbau von Wundauflagen aus zwei funktionalen Schichten die durch thermische Laminierung entsteht. Der Spinnvliesstoff mit glatter Oberfläche ist zur Wunde hin orientiert.
Figur 2 zeigt den Mehrschichtaufbau einer ultraschall-verfestigten Vlieswundauflagen, die aus mehreren Lagen von Standard-Hygiene-Vliesstoffen aufgebaut wird.

### Beispiel

Es werden die physikalischen Daten von Wundauflagen verglichen, drei erfindungsgemäße ultraschallverfestigte PP-Wundauflagen/Kompressen (Beispiele -1 bis 3) mit einer thermisch laminierten PP-Wundauflage/Kompresse (Vergleichsbeispiel).

**Tab. 1**

| Aufbau der Materialien: | | | | |
|---|---|---|---|---|
| ***Merkmal*** | ***Vergleichsbeispiel*** | ***Beispiel 1*** | ***Belspiel 2*** | ***Beispiel 3*** |
| Aufbau /Typ; | Thermisch laminiert | Ultraschall-laminiert | Ultraschall-laminiert | Ultraschall-laminiert |
| | 2 Schichten | 4 Schichten | 5 Schichten | 7 Schichten |
| Wundentklebungsschicht | 15 g/m² PP-Spinnvlies | 2 x 20 g/m² PP-Spinnvlies | 2 x 20 g/m² PP-Spinnvlies | 2 x 20 g/m² PP-Spinnvlies |
| Speicher/Verteilerschicht | 100 g/m² PP-Stapelfaservlies | 2 x 20 g/m² PP-Stapelfaservlies | 3 x 20 g/m² PP-Stapelfaservlies | 5 x 20 g/m² PP-Stapelfaservlies |

**Tab. 2**

| Physikalische Eigenschaften: | | | | | |
|---|---|---|---|---|---|
| ***Merkmal/Methode*** | ***Einheit*** | ***Vergleichsbeispiel*** | ***Beispiel 1*** | ***Belspiel 2*** | ***Beispiel 3*** |
| Flächengewicht DIN EN 29073 T1 | g/m² | 115 | 87 | 111 | 162 |
| Dicke DIN EN 29073 T1 | mm | 0,99 | 0,67 | 0,84 | 01,03 |
| Höchstzugkraft längs DIN EN 29073 T3 | N/50 mm | 27,5 | 158 | 188 | 279 |
| Höchstzugkraft quer DIN EN 29073 T3 | N/50 mm | 13 | 65 | 71 | 93 |
| Trennkraft DIN 53357 | N/50 mm | 1,2 | Nb*) | nb*) | nb*) |
| Wasseraufnahme DIN 53923 | g/m² | 850 | 650 | 500 | 570 |
| Biegesteifigkeit längs | cN*cm² | 3,28 | 2,27 | 4,06 | 9,64 |
| Biegesteifigkeit quer | cN*cm² | 0,94 | - | - | - |

| | | | | | |
|---|---|---|---|---|---|
| *) nb = nicht bestimmbar, da die Kompresse/Wundauflage zerreißt, bevor sich die Schichten voneinander trennen | | | | | |

Der Vergleich der Daten zeigt die besonderen Eigenschaften der mittels Ultraschallverfestigung hergestellten Kompresse/Wundauflage gegenüber der herkömmlichen thermisch-laminierten Variante.

Werte für Höchstzugkräfte in Längs- und Querrichtung sind um den Faktor fünf höher als bei der konventionellen Variante.

Weiterer wichtiger Parameter ist die Trennkraft der Schichten. Während bei der thermisch-laminierten Variante die Trennkraft bei nur 1,2 N/50 mm lag, konnte man die Schichten der Ultraschall-Varianten nicht mehr ohne vollständige Zerstörung der Kompresse/Wundauflage trennen.

Diese Eigenschaft ist insbesondere für die medizinische Anwendung von größter Bedeutung, damit die Kompresse bei der Anwendung auf der Wunde nicht spaltet und die obere Wundentklebungsschicht möglicherweise in der Wunde verbleibt.
Die Wasseraufnahme ist für Ultraschall-Varianten leicht emiedrigt. Die Ursache ist im unterschiedlichen Schichten-Aufbau begründet: Es handelt sich um einen Verbund aus mehreren Schichten in der Speicherschicht, die jede für sich genommen in sich stärker verfestigt sind als ein Material, daß nur aus einer Speicherschicht besteht. Der höhere Verfestigungsgrad der einzelnen Vliesschichten führt dadurch zu einer emiedrigten Flüssigkeitsaufnahme im fertigen Produkt.

## Patentansprüche

1. Wundauflage und/oder Kompresse aus einer der Haut zugewandten Lage eines Spinnvliesstoffes sowie mindestens einer auf dem Spinnvliesstoff angeordneten Lage eines Stapelfaservliesstoffes, wobei die Lagen unlösbar durch Ultraschall-Verschweißen miteinander verbunden sind.

2. Wundauflage und/oder Kompresse nach Anspruch 1, **dadurch gekennzeichnet, daß** der Spinnvliesstoff und der Stapelfaservliesstoff aus Polypropylen bestehen.

3. Wundauflage und/oder Kompresse nach Anspruch 1 und 2, **dadurch gekennzeichnet, daß** der Stapelfaservliesstoff ein Flächengewicht von 15 bis 35 g/m² und/oder der Spinnvliesstoff ein Flächengewicht von 8 bis 25 g/m² aufweist.

4. Wundauflage und/oder Kompresse nach einem der Ansprüche 1 bis 3, **dadurch gekennzeichnet, daß** das Trägermaterial bis zu fünf Lagen des Stapelfaservliesstoffes umfaßt.

5. Wundauflage und/oder Kompresse nach einem der Ansprüche 1 bis 4, **dadurch gekennzeichnet, daß** der Spinnvliesstoff
mit Wirkstoffen dotiert und/oder
mit bakteriziden und/oder fungizider Stoffen und/oder
mit mikroverkapselten kosmetischen Wirkstoften ausgerüstet ist.

6. Wundauflage und/oder Kompresse nach einem der Ansprüche 1 bis 5, **dadurch gekennzeichnet, daß** den Spinnvliesstoffen hochfeste Fasern, Zwirne, Mischzwirne oder Fäden mit einer Höchstzugkraft von mindestens 60 cN/tex zugesetzt sind.

7. Verfahren zur Herstellung eines einer Wundauflage und/oder Kompresse nach Anspruch 7, **dadurch gekennzeichnet, daß** die Verschweißpunkte einen Abstand zwischen 2 und 5 mm in Längs- und Querrichtung aufweisen, insbesondere 3 mm bis 4 mm.

8. Wundauflage und/oder Kompresse aus einer der Haut zugewandten Lage einer perforierten Folie sowie mindestens einer auf der Folie angeordneten Lage eines Vliesstoffes, wobei die Lagen unlösbar durch Ultraschallverschweißen miteinander verbunden sind.

9. Wundauflage und/oder Kompresse nach Anspruch 6, **dadurch gekennzeichnet, daß** die Folie aus Polypropylen besteht.

10. Wundauflage und/oder Kompresse nach den Ansprüchen 8 und 9, **dadurch gekennzeichnet, daß** auf dem Vliesstoff mindestens eine Lage eines Spinnvliesstoffes vorhanden ist, wobei die Lagen unlösbar miteinander verbunden sind.
